# EUROPEAN PATENT SPECIFICATION

(11) **EP 3 171 862 B1**
(45) Date of publication and mention of the grant of the patent: **05.07.2023**
(21) Application number: 15741210.7
(22) Date of filing: 23.07.2015
(51) Int. Cl.: C12N 11/04, A61K 9/50, G01N 33/66, B05B 7/16, C12N 9/04, C12Q 1/00

(54) **METHOD AND APPARATUS FOR GENERATION OF MICROPARTICLES CONTAINING IMMOBILIZED ENZYME**
VERFAHREN UND VORRICHTUNG ZUR ERZEUGUNG VON MIKROPARTIKELN MIT IMMOBILISIERTEN ENZYMEN
PROCÉDÉ ET APPAREIL POUR LA GÉNÉRATION DE MICROPARTICULES CONTENANT UNE ENZYME IMMOBILISÉE

(30) Priority: 23.07.2014 EP 14178121
(43) Date of publication of application: 31.05.2017
(73) Proprietor: Roche Diabetes Care GmbH, 68305 Mannheim (DE); F. Hoffmann-La Roche AG, 4070 Basel (CH)
(72) Inventor: FUERST, Otto, 68519 Viernheim (DE); HARTTIG, Herbert, 67434 Neustadt (DE); HILLER, Bernd, 68623 Lampertheim (DE)
(74) Representative: Pfiz, Thomas
(86) International application number: PCT/EP2015/066936
(87) International publication number: WO 2016/012564

(56) References cited:
- EP-A1- 0 162 302
- US-A1- 2010 035 245
- KENG-SHIANG HUANG ET AL: "Electrostatic droplets assisted synthesis of alginate microcapsules", DRUG DELIVERY AND TRANSLATIONAL RESEARCH, vol. 1, no. 4, 22 March 2011 (2011-03-22), pages 289-298, XP055160141, ISSN: 2190-393X, DOI: 10.1007/s13346-011-0020-8
- HIDEO WATANABE ET AL: "Preparation of immobilized enzyme gel particles using an electrostatic atomization technique", BIOCHEMICAL ENGINEERING JOURNAL, vol. 8, no. 2, 1 September 2001 (2001-09-01), pages 171-174, XP055160389, ISSN: 1369-703X, DOI: 10.1016/S1369-703X(01)00121-8
- HERRERO E P ET AL: "Development of a new technology for the production of microcapsules based in atomization processes", CHEMICAL ENGINEERING JOURNAL,, vol. 117, no. 2, 1 April 2006 (2006-04-01) , pages 137-142, XP028035830, ISSN: 1385-8947, DOI: 10.1016/J.CEJ.2005.12.022 [retrieved on 2006-04-01]

## Description

### Description

The invention concerns a method and an apparatus for generation of microparticles containing an immobilized functional component, specifically an enzyme or other functional molecules or sub-micro-sized particles (nanoparticles) as defined by the claims. The invention further concerns a diagnostic test element, specifically glucose test element as defined by the claims.

In diagnostic test elements or sensors for glucose tests it is known to provide enzymes in a reagent layer or electrode layer to induce chemical reactions which are responsive to an analyte in a body fluid contacted with the test element. Such analyses are usually made with handheld devices on the spot by patients themselves. There, it should be guaranteed that the enzymes are immobilized without skin contact, and that the enzymes can be reached by diffusion of the sample ingredients in a given measurement time. In this context, it is known to include the enzymes in a wet chemistry composition which is applied on a substrate and further coated after drying.

The research article KENG-SHIANG HUANG ET AL, "Electrostatic droplets assisted synthesis of alginate microcapsules", DRUG DELIVERY AND TRANSLATIONAL RESEARCH, (20110322), vol. 1, no. 4, , 22 March 2011, pages 289-298 proposes an Electrostatic Droplet Generating System (ESD) to pump an alginate solution through a syringe needle where high voltage is applied such that the body of the liquid becomes charged and is split after passing the needle tip to only then form a series of isolated droplets.

EP 0 162 302 A1 only discloses titanium dioxide microparticles for forming a light shielding layer in an integral multilayer analytical element.

On this basis the object of the invention is to further improve the known methods and for generation of microparticles and to provide improved diagnostic test elements specifically for self-testing glucose measurement systems.

The combination of features stated in the independent claims is proposed to achieve this object. Advantageous embodiments and further developments of the invention are derived from the dependent claims.

The invention is based on the idea of generating solidified microparticles as a carrier for at least one immobilized functional component, which has a specific function in a diagnostic test. Accordingly it is proposed according to the invention that a method for generation of microparticles comprises the steps of
a) spraying a liquid which contains a soluble alginate and a functional component consisting of molecules or nanoparticles to generate a stream of droplets, wherein spraying the liquid comprises applying a gas stream to the liquid in a reservoir, thereby atomizing the liquid in the gas stream,
b) directing the stream of previously generated droplets through an electrically conductive nozzle which is connected as an electrode to high voltage, such that electrostatic charging of the droplets occurs during passage of the nozzle,
c) directing the stream of droplets onto a precipitation bath and capturing the droplets therein by application of the high voltage,
d) precipitating the droplets in the precipitation bath by means of a precipitation liquid containing an alginate complexing agent, whereby the droplets are solidified to form microparticles containing the functional component, and
e) extracting the microparticles from the precipitation bath.

In such a way, it is possible to create particles from a physiological non-hazardous material and of very small size, i.e. at most 100 microns, so that there is provided a comparatively large surface for the analytes to reach the functional component which is immobilized in the particles structure. An adequate particle size can be achieved by atomizing a liquid and by directing a mist-like stream of droplets under the influence of a high voltage to a precipitation bath, such that a considerable yield is achieved and the spray is not lost before reaching the precipitation liquid. By atomizing the liquid in the gas stream, there is no need for a complex electrostatic atomization technique. Moreover, such a gas stream allows adjustment of the droplet size by simple measures such as adjusting the velocity and temperature of the carrier gas. Thus, the generation of a stream of droplets in a gas stream provides further degrees of freedom: The droplet size can be adjusted independent of the voltage of a potential subsequent electrostatic charge. By means of increasing the gas pressure, it is possible to decrease the average diameter of the droplets, even below a dimension which is achievable by electrostatic atomization of a given liquid, e.g. to 2-10 µm. The droplet dimension is not influenced, neither by the distance to the precipitation bath nor by the electric conductivity of the initial solution nor by the magnitude of an applied high voltage. Furthermore, it is possible to achieve a high throughput with simple measures. Surprisingly, the electrostatic charging of the droplets within the gas stream during passage of the high voltage nozzle works without problems and efficiently. Without the need for a direct contact with the nozzle, an electrostatic charging of the droplets occurs, which is used to bring the droplets in effective contact with the precipitation bath. Due to the electric charge, an attractive force occurs in direction to the inversely poled precipitation bath, which brings the micro-droplets in contact with the bath surface.

According to a preferred embodiment, the molecules forming the functional component are selected from the group of enzymes, coenzymes, mediators, stabilizers and dyes, and preferably are enzymes.

It is also preferred that the nanoparticles forming the functional component are sized in at least one dimension, preferably in 3 dimensions, below 1 µm. The nanoparticles may preferably be selected from the group of metal, metal alloy, metal oxide and carbon.

For further improvement of the yield of generated particles, it is advantageous when a moving surface is provided in the precipitation bath, wherein the moving surface is continuously loaded with a film of the precipitation liquid and the droplets are disposed onto the moving surface such that agglutination of droplets or particles is avoided.

Another improvement provides that a target electrode connected to the high voltage is arranged in the precipitation bath, specifically in the form of a submerged rotating drum. This allows to provide a focused attractive force in addition to gravitation.

In a particular embodiment the microparticles are formed with a dimension of less than 50 µm, e.g. in the range from 1 to 20 µm, preferably less than 20 µm, e.g. in the range from 1 to 10 µm, and more preferably less than 10 µm, e.g. in the range from 0.1 to 5 µm. Microparticles of such a small size provide sufficient surface as a diffusion interface, and with a limited size distribution preferably in the single-digit micron range it is possible to achieve a homogenous response.

Advantageously, the method further comprises the step of separating microparticles of different size by an ion exchange process using different ions in the alginate complexing agent, specifically barium ions and calcium ions.

For further improvement of the practical value it is advantageous when the microparticles are provided with a stabilizing shell made of a polymer material, specifically of polycations.

An intermediate product can be generated for further processing by discharging a suspension of the microparticles through an outlet of the precipitation bath.

It is also favorable when the microparticles are treated in a cleaning bath by means of an ion exchange process, specifically to remove biological hazardous substances.

In regard to a preferred use it is advantageous when the microparticles are deposited in a layer of a diagnostic test element, specifically in a reagent layer or electrode layer of a glucose test element.

With regard to an apparatus adapted for generation of microparticles containing an immobilized functional component, in order to solve the aforementioned object, it is proposed to provide a modular arrangement comprising the following components:
- a spraying unit adapted to generate a stream of droplets of a liquid containing a soluble alginate and the functional component, wherein the stream of droplets is generated by applying a gas stream to the liquid in a reservoir, thereby atomizing the liquid in the gas stream,
- wherein an electrically conductive nozzle is connected as a counter electrode to the high voltage unit, and the stream of previously generated droplets is directed through the nozzle,
- a high voltage unit adapted for charging and directing the droplets to a target electrode and
- a precipitation bath containing the target electrode and an alginate complexing agent adapted to precipitate the droplets, such that the droplets are solidified to form microparticles,
- means adapted to extract the microparticles from the precipitation bath.

Another aspect of the invention concerns a diagnostic test element, specifically glucose test element, comprising a reagent layer or electrode layer, wherein said layer comprises microparticles containing at least one immobilized functional component which consists of nanoparticles, wherein the nanoparticles forming the functional component are selected of at least one of platinum, palladium, silver, rhenium, rhodium, iron, nickel, cobalt, copper, chromium, zinc, aluminium, manganese and molybdenum or alloys thereof or oxides thereof.

The proposed inclusion of microparticles is particularly effective when a two-layer structure comprising an intermediate and an outer layer is applied on a carrier substrate, and when the microparticles are included in the intermediate layer.

Further advantageously and specifically tailor-made for blood glucose tests, the at least one functional component is an enzyme which is selected from the following group of enzymes: oxi-reductase-enzymes, e.g. GlucDOR/PQQ (Glucose Dye Oxidoreductase), dehydrogenase-enzymes, e.g. glucose dehydrogenase, in particular NAD-dependent glucose dehydrogenase, FAD-dependent glucose dehydrogenase and/or PQQ-dependent glucose dehydrogenase, oxidase-enzymes, e.g. glucose oxidase.

Generally, the sub-micro-sized particles or nanoparticles forming the functional component are considerably smaller than the microparticles and have a diameter of less than 1 µm, preferably 1 to 500 nm, especially preferred of 1 to 100 nm.

The invention is further elucidated in the following on the basis of an embodiment example shown schematically in the drawings, where
Fig. 1 is a schematic view of an apparatus for generation of microparticles;
Fig. 2 is sectional view of a layered diagnostic test element comprising microparticles.

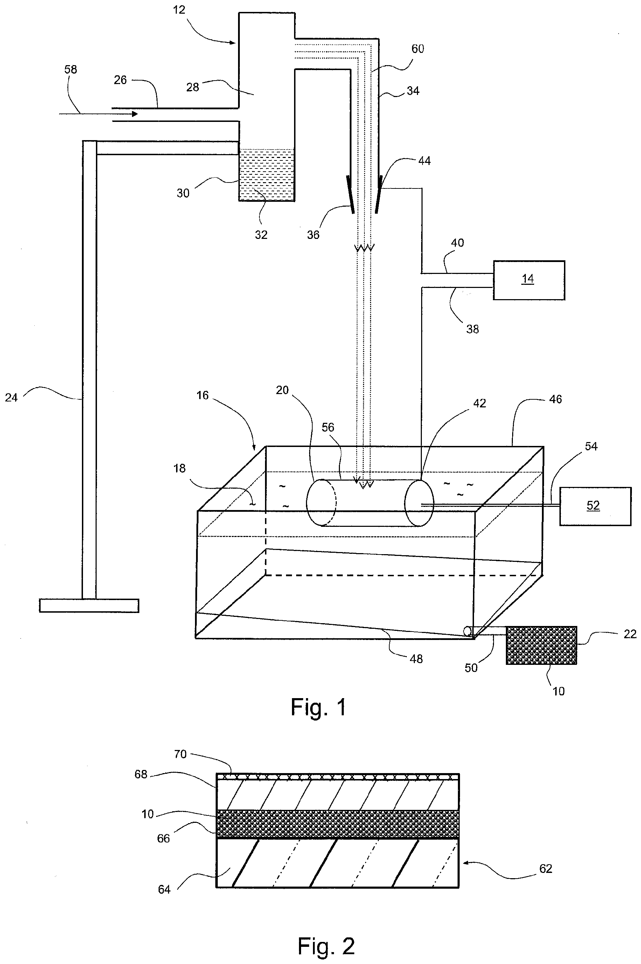

Fig. 1 illustrates a system or apparatus for generation of microparticles 10 comprising a spraying unit 12, a high voltage unit 14, a precipitation bath 16 including a precipitation liquid 18 and a rotatable drum 20, and extraction means 22 for extracting precipitated microparticles 10 from the bath 16.

The spraying unit 12, which can be positioned over the precipitation bath 16 by means of a stand 24, is provided with a gas inlet 26, an atomizer chamber 28 including a reservoir 30 for a feed liquid 32 and an aerosol outlet 34 ending in a discharge nozzle 36. The feed liquid 32 contains an aqueous solution of a soluble alginate, e.g. sodium alginate and at least one of enzyme selected from oxi-reductase-enzymes, dehydrogenase-enzymes or oxidase-enzymes and other functional molecules. Therewith, it is finally aimed to form beads of alginate complexes containing immobilized enzymes as (spherical) microparticles with a size or dimension (diameter) in the range from 1 to 20 µm.

The high voltage unit 14 has a source 38 for a d.c. voltage in the range of 3 to 80 kV, preferred 10 to 60 kV which is supplied between a first port 38 and a second port 40. First port 38, suitably grounded, is connected to the drum 20 which thereby forms a target electrode 42, whereas second port 40 is connected to the nozzle 36 forming a counter electrode 44. Optionally, the first port 38 may be connected to the precipitation liquid 18, and second port 40 may be connected to charge the feed liquid 32.

The precipitation bath 16 comprises an open-top container 46 which has an inclined bottom plane 48 for guiding precipitated microparticles 10 to a discharge connection 50 of the extraction means 22.

The horizontally oriented drum 20 is rotatable around its center axis by means of a motor 52, where the output shaft 54 is arranged along the fluid level of the precipitation liquid 18. In this way, the cylindrical mantle of the rotated drum 20 is partially submersed and hence forms a moving surface 56 which is continuously loaded with a revolving film of the precipitation liquid 18.

The system may comprise a central control unit (not shown) for controlling the operating procedure and the process parameters of the various system units.

In use, the inlet 26 of the spraying unit 12 is loaded with a stream 58 of a carrier gas, e.g. air provided from a compressor. Under the effect of the gas stream 58, the feed liquid 32 is atomized into nebular droplets. Advantageously, the size of the droplets is adjusted by the velocity, temperature and humidity of the carrier gas, wherein evaporation leads to a miniaturization of the droplets during flight. In this stage, a fine aerosol jet 60 is generated by non-electric effects.

Then, the jet 60 is ejected through the nozzle 36, where the droplets are electrically charged to a high potential. The nozzle 36 directs the jet 60 against the drum 20, where the charged droplets are attracted by the target electrode 42 and captured in the film of the precipitation liquid 18 coating the mantle or moving surface 56.

The precipitation liquid 18 contains an aqueous solution of an alginate complexing agent including e.g. Ba²⁺ ions which permeate the droplets and lead to solidified beads or microparticles 10 containing immobilized enzyme(s).

In addition to the complexing agent, further ingredients may be provided in the precipitation liquid 18 to structurally stabilize the developing beads on their surface. This may be achieved by polymers, specifically polycations, which preferentially adsorb on the surface of the beads and form a complex with the solidifying alginate component thereby providing a stabilizing outer shell.

The solidified beads which sediment on the inclined bottom plane 48 are guided to the discharge connection 50, where a suspension of microparticles 10 can be discharged to the extraction means 22 either in a continuous or a batch mode.

It is also conceivable to separate microparticles of different size by a cation ion exchange process. When the microparticles 10, which were precipitated with ions of comparatively high atomic mass, are fed into a solution of a precipitating ion of lower atomic mass, e.g. Ca²⁺ in a saturated CaSO₄-solution, an ion exchange process occurs with the resulting beads having a lower density. As this happens faster on smaller particles having a comparatively larger surface, smaller beads will ascend in the solution, and a separation can be achieved by decantation. At the same time, the ion exchange process leads to a cleaning in the sense of a reduced toxicity of Ca²⁺-containing microparticles 10.

The microparticles 10 containing immobilized enzyme(s) are particularly useful in diagnostic test elements designed for glucose tests. Such test elements may be provided on disposable test tapes or test strips, either for optical or electrochemical analyses.

Fig. 2 shows a sectional view of a glucose test element 62 which is generally designed as a two-layered composite on a substrate 62 formed by a transparent plastic carrier. The substrate 62 is coated with a first layer 66 of a reactive test material. This material contains microparticles 10 including immobilized enzymes and some of functional molecules as mediator and dye. The latter reacts by a color change induced by the enzymes which are responsive to glucose. In one exemplary composition, oxidase- or dehydrogenase enzymes are used, e.g. glucose oxidase or glucose dehydrogenase, and the dye includes molybdenum in the form of phosphomolybdic acid.

The first layer 66 is covered by a second layer 68 of test material containing most of the mediator and of the dye which are also present in the first layer. Further, the second layer 68 contains white pigments for separation of a blood sample and for providing a white background for optical measurement of the color change. It is notably important to avoid that enzymes and other functional molecules permeate the optical barrier formed by the second layer 68 specifically during drying of the respective wet chemistry composition. The microparticles 10 immobilize the enzymes in such a way that they cannot reach the second layer 68 during the manufacturing or analysis process.

On the upper side of the test element 62, a spreading web 70 is attached for homogenous and planar distribution of a blood sample. When conducting a diagnostic test, the blood sample is applied by the user as a droplet from a skin wound.

## Claims

1. A method for generation of microparticles (10) containing an immobilized functional component, the method comprising the steps of
a) spraying a liquid (32) which contains a soluble alginate and a functional component consisting of molecules or nanoparticles to generate a stream (60) of droplets, wherein spraying the liquid comprises applying a gas stream (58) to the liquid (32) in a reservoir (30), thereby atomizing the liquid in the gas stream,
b) directing the stream (60) of previously generated droplets through an electrically conductive nozzle (36) which is connected as an electrode (44) to high voltage (14), such that electrostatic charging of the droplets occurs during passage of the nozzle (36),
c) directing the stream (60) of droplets onto a precipitation bath (16) and capturing the droplets therein by application of the high voltage (14),
d) precipitating the droplets in the precipitation bath (16) by means of a precipitation liquid (18) containing an alginate complexing agent, such that the droplets are solidified to form microparticles (10) containing the functional component, and
e) extracting the microparticles (10) from the precipitation bath (16).

2. The method of claim 1, wherein the molecules forming the functional component are selected from the group of enzymes, coenzymes, mediators, stabilizers and dyes, and preferably are enzymes, and/or wherein the nanoparticles forming the functional component are sized in at least one dimension below 1 µm and/or are selected from the group of metal, metal alloy, metal oxide and carbon.

3. The method of claim 1 or 2, further comprising
providing a moving surface (56) in the precipitation bath (16), wherein the moving surface (56) is continuously loaded with a film of the precipitation liquid (18) and the droplets are disposed onto the moving surface (56).

4. The method according to any of claims 1 to 3, wherein a target electrode (42) connected to the high voltage (14) is arranged in the precipitation bath (16), specifically in the form of a submerged rotating drum (20).

5. The method according to any of claims 1 to 4, wherein the microparticles (10) are formed with a dimension of less than 50 µm, preferably in the range from 1 to 20 µm.

6. The method according to any of claims 1 to 5, further comprising separating microparticles (10) of different size by an ion exchange process using different ions in the alginate complexing agent, specifically barium ions and calcium ions.

7. The method according to any of claims 1 to 6, further comprising providing the microparticles (10) with a stabilizing shell made of a polymer material.

8. The method according to any of claims 1 to 7, further comprising discharging a suspension of the microparticles (10) through an outlet (50) of the precipitation bath (16).

9. The method according to any of claims 1 to 8, further comprising cleaning of the microparticles (10) in a cleaning bath by means of an ion exchange process.

10. The method according to any of claims 1 to 9, further comprising depositing the microparticles (10) in a layer (66) of a diagnostic test element (62), specifically in a reagent layer or electrode layer of a glucose test element.

11. An apparatus for generation of microparticles (10) containing an immobilized functional component, wherein the functional component consists of molecules or nanoparticles, comprising
a) a spraying unit (12) adapted to generate a stream (60) of droplets of a liquid (32) containing a soluble alginate and the functional component, wherein the stream (60) of droplets is generated by applying a gas stream (58) to the liquid (32) in a reservoir (30), thereby atomizing the liquid in the gas stream,
b) a high voltage unit (14) for directing the droplets to a target electrode (42),
c) wherein an electrically conductive nozzle (36) is connected as a counter electrode (44) to the high voltage unit (14), and the stream (60) of previously generated droplets is directed through the nozzle (36),
d) a precipitation bath (16) containing the target electrode (42) and an alginate complexing agent adapted to precipitate the droplets, such that the droplets are solidified to form microparticles (10), and
e) means (50) adapted to extract the microparticles (10) from the precipitation bath (16).

12. Diagnostic test element, specifically glucose test element, comprising a reagent layer (66) or electrode layer, wherein said layer (66) comprises microparticles (10) containing at least one immobilized functional component which consists of nanoparticles, wherein the nanoparticles forming the functional component are selected of at least one of platinum, palladium, silver, rhenium, rhodium, iron, nickel, cobalt, copper, chromium, zinc, aluminium, manganese and molybdenum or alloys thereof or oxides thereof, wherein the microparticles are generated by a method according to any of claims 1 to 10.

13. The diagnostic test element of claim 12, wherein a two-layer structure comprising an intermediate and an outer layer (66,68) is applied on a carrier substrate (64), and the microparticles (10) are included in the intermediate layer (66).

## Patentansprüche

1. Verfahren zur Erzeugung von eine immobilisierte funktionelle Komponente enthaltenden Mikropartikeln (10), wobei das Verfahren die folgenden Schritte umfasst
a) Sprühen einer Flüssigkeit (32), die ein lösliches Alginat und eine aus Molekülen oder Nanopartikeln bestehende funktionelle Komponente enthält, um einen Strom (60) von Tröpfchen zu erzeugen, wobei das Sprühen der Flüssigkeit das Aufbringen eines Gasstroms (58) auf die Flüssigkeit (32) in einem Reservoir (30) umfasst, wodurch die Flüssigkeit in dem Gasstrom zerstäubt wird,
b) Leiten des Stroms (60) von zuvor erzeugten Tröpfchen durch eine elektrisch leitende Düse (36), die als Elektrode (44) an Hochspannung (14) angeschlossen ist, so dass eine elektrostatische Aufladung der Tröpfchen während des Durchgangs durch die Düse (36) erfolgt,
c) Leiten des Stroms (60) von Tröpfchen auf ein Fällungsbad (16) und Einfangen der Tröpfchen darin durch Anlegen der Hochspannung (14),
d) Ausfällen der Tröpfchen in dem Fällungsbad (16) mittels einer einen Alginatkomplexbildner enthaltenden Fällungsflüssigkeit (18), so dass die Tröpfchen zu Mikropartikeln (10) verfestigt werden, die die funktionelle Komponente enthalten, und
e) Extrahieren der Mikropartikel (10) aus dem Fällungsbad (16).

2. Verfahren nach Anspruch 1, wobei die die funktionelle Komponente bildenden Moleküle aus der Gruppe der Enzyme, Coenzyme, Mediatoren, Stabilisatoren und Farbstoffe ausgewählt sind und vorzugsweise Enzyme sind, und/oder wobei die die funktionelle Komponente bildenden Nanopartikel in mindestens einer Dimension unter 1 µm groß sind und/oder aus der Gruppe der Metalle, Metalllegierungen, Metalloxide und Kohlenstoff ausgewählt sind.

3. Verfahren nach Anspruch 1 oder 2, ferner umfassend
Bereitstellen einer beweglichen Oberfläche (56) in dem Fällungsbad (16), wobei die bewegliche Oberfläche (56) kontinuierlich mit einem Film der Fällungsflüssigkeit (18) beladen wird und die Tröpfchen auf der beweglichen Oberfläche (56) abgesetzt werden.

4. Verfahren nach einem der Ansprüche 1 bis 3, wobei im Fällbad (16) eine an die Hochspannung (14) angeschlossene Targetelektrode (42) angeordnet ist, insbesondere in Form einer eingetauchten rotierenden Trommel (20).

5. Verfahren nach einem der Ansprüche 1 bis 4, wobei die Mikropartikel (10) mit einer Größe von weniger als 50 µm, vorzugsweise im Bereich von 1 bis 20 µm, gebildet werden.

6. Verfahren nach einem der Ansprüche 1 bis 5, ferner umfassend die Abtrennung von Mikropartikeln (10) unterschiedlicher Größe durch ein Ionenaustauschverfahren unter Verwendung unterschiedlicher Ionen im Alginatkomplexbildner, insbesondere Bariumionen und Calciumionen.

7. Verfahren nach einem der Ansprüche 1 bis 6, umfassend ferner das Bereitstellen der Mikropartikel (10) mit einer stabilisierenden Hülle aus einem Polymermaterial.

8. Verfahren nach einem der Ansprüche 1 bis 7, ferner umfassend das Austragen einer Suspension der Mikropartikel (10) durch einen Auslass (50) des Fällungsbades (16).

9. Verfahren nach einem der Ansprüche 1 bis 8, ferner umfassend das Reinigen der Mikropartikel (10) in einem Reinigungsbad mittels eines Ionenaustauschprozesses.

10. Verfahren nach einem der Ansprüche 1 bis 9, wobei die Mikropartikel (10) in einer Schicht (66) eines diagnostischen Testelements (62), insbesondere in einer Reagenzschicht oder Elektrodenschicht eines Glukosetestelements, abgelagert werden.

11. Vorrichtung zur Erzeugung von eine immobilisierte funktionelle Komponente enthaltenden Mikropartikeln (10), wobei die funktionelle Komponente aus Molekülen oder Nanopartikeln besteht, umfassend
a) eine Sprüheinheit (12), die ausgebildet ist, einen Strom (60) von Tröpfchen einer Flüssigkeit (32) zu erzeugen, die ein lösliches Alginat und die funktionelle Komponente enthält, wobei der Strom (60) von Tröpfchen erzeugt wird, indem ein Gasstrom (58) auf die Flüssigkeit (32) in einem Reservoir (30) aufgebracht wird, wodurch die Flüssigkeit in dem Gasstrom zerstäubt wird,
b) eine Hochspannungseinheit (14) zum Lenken der Tröpfchen auf eine Zielelektrode (42),
c) wobei eine elektrisch leitende Düse (36) als Gegenelektrode (44) an die Hochspannungseinheit (14) angeschlossen ist und der Strom (60) der zuvor erzeugten Tröpfchen durch die Düse (36) geleitet wird,
d) ein Fällungsbad (16), das die Zielelektrode (42) und einen Alginatkomplexbildner enthält, der geeignet ist, die Tröpfchen auszufällen, so dass die Tröpfchen zu Mikropartikeln (10) verfestigt werden, und
e) Mittel (50), die geeignet sind, die Mikropartikel (10) aus dem Fällungsbad (16) zu extrahieren.

12. Diagnostisches Testelement, insbesondere Glukose-Testelement, umfassend eine Reagenzschicht (66) oder Elektrodenschicht, wobei die Schicht (66) Mikropartikel (10) umfasst, die mindestens eine immobilisierte funktionelle Komponente enthalten, die aus Nanopartikeln besteht, wobei die die funktionelle Komponente bildenden Nanopartikel ausgewählt sind aus wenigstens einem von Platin, Palladium, Silber, Rhenium, Rhodium, Eisen, Nickel, Kobalt, Kupfer, Chrom, Zink, Aluminium, Mangan und Molybdän oder Legierungen davon oder Oxiden davon, wobei die Mikropartikel nach einem Verfahren nach einem der Ansprüche 1 bis 10 erzeugt werden.

13. Diagnostisches Testelement nach Anspruch 12, wobei auf einem Trägersubstrat (64) eine zweischichtige Struktur mit einer Zwischen- und einer Außenschicht (66, 68) aufgebracht ist, und die Mikropartikel (10) in der Zwischenschicht (66) enthalten sind.

## Revendications

1. Procédé pour la production de microparticules (10) contenant un constituant fonctionnel immobilisé, le procédé comprenant les étapes
a) de pulvérisation d'un liquide (32) qui contient un alginate soluble et un constituant fonctionnel constitué de molécules ou de nanoparticules afin de produire un courant (60) de gouttelettes, dans lequel la pulvérisation du liquide comprend l'application d'un courant gazeux (58) au liquide (32) dans un réservoir (30), atomisant de ce fait le liquide dans le courant gazeux,
b) de direction du courant (60) de gouttelettes précédemment produites à travers une buse électriquement conductrice (36) qui est connectée en tant qu'électrode (44) à une haute tension (14), de telle sorte que la charge électrostatique des gouttelettes se produise durant le passage dans la buse (36),
c) de direction du courant (60) de gouttelettes sur un bain de précipitation (16) et de capture des gouttelettes en son sein par application de la haute tension (14),
d) de précipitation des gouttelettes dans le bain de précipitation (16) au moyen d'un liquide de précipitation (18) contenant un agent complexant de l'alginate, de telle sorte que les gouttelettes soient solidifiées afin de former des microparticules (10) contenant le constituant fonctionnel, et
e) d'extraction des microparticules (10) du bain de précipitation (16).

2. Procédé selon la revendication 1, dans lequel les molécules formant le constituant fonctionnel sont sélectionnées dans le groupe des enzymes, des coenzymes, des médiateurs, des stabilisants et des colorants, et de préférence sont des enzymes, et/ou dans lequel les nanoparticules formant le constituant fonctionnel sont dimensionnées en au moins une dimension au-dessous de 1 µm et/ou sont sélectionnées dans le groupe du métal, d'alliage métallique, d'oxyde métallique et du carbone.

3. Procédé selon la revendication 1 ou la revendication 2, comprenant en outre la fourniture d'une surface mouvante (56) dans le bain de précipitation (16), dans lequel la surface mouvante (56) est chargée en continu d'un film du liquide de précipitation (18) et les gouttelettes sont disposées sur la surface mouvante (56).

4. Procédé selon l'une quelconque des revendications 1 à 3, dans lequel une électrode cible (42) connectée à la haute tension (14) est agencée dans le bain de précipitation (16), spécifiquement sous la forme d'un tambour rotatif immergé (20).

5. Procédé selon l'une quelconque des revendications 1 à 4, dans lequel les microparticules (10) sont formées avec une dimension de moins de 50 µm, de préférence comprise dans la gamme allant de 1 à 20 µm.

6. Procédé selon l'une quelconque des revendications 1 à 5, comprenant en outre la séparation de microparticules (10) de tailles différentes par un processus d'échange d'ions utilisant des ions différents dans l'agent complexant de l'alginate, spécifiquement des ions baryum et des ions calcium.

7. Procédé selon l'une quelconque des revendications 1 à 6, comprenant en outre la fourniture aux microparticules (10) d'une coquille stabilisante réalisée en un matériau polymère.

8. Procédé selon l'une quelconque des revendications 1 à 7, comprenant en outre le déversement d'une suspension des microparticules (10) par une sortie (50) du bain de précipitation (16).

9. Procédé selon l'une quelconque des revendications 1 à 8, comprenant en outre le nettoyage des microparticules (10) dans un bain de nettoyage au moyen d'un processus d'échange d'ions.

10. Procédé selon l'une quelconque des revendications 1 à 9, comprenant en outre le dépôt des microparticules (10) dans une couche (66) d'un élément de test diagnostique (62), spécifiquement dans une couche de réactif ou couche d'électrode d'un élément de test de glucose.

11. Appareil pour la production de microparticules (10) contenant un constituant fonctionnel immobilisé, dans lequel le constituant fonctionnel est constitué de molécules ou de nanoparticules, comprenant
a) une unité de pulvérisation (12) adaptée à produire un courant (60) de gouttelettes d'un liquide (32) contenant un alginate soluble et le constituant fonctionnel, dans lequel le courant (60) de gouttelettes est produit par application d'un courant gazeux (58) au liquide (32) dans un réservoir (30), atomisant de ce fait le liquide dans le courant gazeux,
b) une unité haute tension (14) pour diriger les gouttelettes vers une électrode cible (42),
c) dans lequel une buse électriquement conductrice (36) est connectée en tant que contre-électrode (44) à l'unité haute tension (14), et le courant (60) de gouttelettes précédemment produites est dirigé à travers la buse (36),
d) un bain de précipitation (16) contenant l'électrode cible (42) et un agent complexant de l'alginate adapté à précipiter les gouttelettes, de telle sorte que les gouttelettes soient solidifiées afin de former des microparticules (10), et
e) des moyens (50) adaptés à extraire les microparticules (10) du bain de précipitation (16).

12. Élément de test diagnostique, spécifiquement élément de test de glucose, comprenant une couche de réactif (66) ou couche d'électrode, dans lequel ladite couche (66) comprend des microparticules (10) contenant au moins un constituant fonctionnel immobilisé qui est constitué de nanoparticules, dans lequel les nanoparticules formant le constituant fonctionnel sont sélectionnées parmi au moins un élément parmi le platine, le palladium, l'argent, le rhénium, le rhodium, le fer, le nickel, le cobalt, le cuivre, le chrome, le zinc, l'aluminium, le manganèse et le molybdène ou des alliages de ceux-ci ou des oxydes de ceux-ci, dans lequel les microparticules sont produites par un procédé selon l'une quelconque des revendications 1 à 10.

13. Élément de test diagnostique selon la revendication 12, dans lequel une structure à deux couches comprenant une couche intermédiaire et une couche externe (66, 68) est appliquée sur un substrat de support (64), et les microparticules (10) sont incluses dans la couche intermédiaire (66).
